Europäisches Patentamt

**European Patent Office**

**Office européen des brevets**

(11) Publication number: **0 134 868**
**A1**

(12) **EUROPEAN PATENT APPLICATION**

(21) Application number: **83401713.9**

(22) Date of filing: **26.08.83**

(51) Int. Cl.⁴: **G 01 N 33/53**
**C 07 K 15/04**

(43) Date of publication of application:
**27.03.85 Bulletin 85/13**

(84) Designated Contracting States:
**AT BE CH DE FR GB IT LI NL SE**

(71) Applicant: **ANDA BIOLOGICALS**
**37, rue de la Course**
**F-67000 Strasbourg(FR)**

(72) Inventor: **Maes, Roland F.**
**10A, rue du 22 Novembre**
**F-67000 Strasbourg(FR)**

(74) Representative: **Portal, Gérard et al,**
**Cabinet Z. Weinstein 20, Avenue de Friedland**
**F-75008 Paris(FR)**

(54) **Unicellular organisms activated by glutaraldehyde as solid carriers for sensitizing agents and uses of sensitized unicellular organisms.**

(57) Bacteria, yeast cells, red blood cells and other unicellular entities are used as a solid support or carrier for the attachment of sensitizing agents such as antigens, antibodies, nucleic acids, amino-acids, haptens, immunogens, receptors and the like. The coupling agent consists in a glutaraldehyde solution at 2.5%. Sensitized unicellular organisms are useful in immunizing procedures, in diagnostic tests based on the use of a sensitized solid phase and in affinity separations.

## UNICELLULAR ORGANISMS ACTIVATED BY GLUTARALDEHYDE AS SOLID CARRIERS FOR SENSITIZING AGENTS AND USES OF SENSITIZED UNICELLULAR ORGANISMS

This invention relates to an improvement in methods for the isolation, detection and determination of peptides, antigens, nucleic acids, immunogens, antibodies, binding partners and the like as well as for the formation of antibodies in vivo.

A number of methods have been developed for the isolation and determination of antigens and antibodies which rely on the attachment of one of the binding partners to a solid phase. A sensitized solid carrier is used either in order to facilitate the manipulations and the isolation of one of the binding partners engaged in the determination or isolation, or in order to provide a visual indication that the binding reaction has taken place.

In radio-immunoassay and in enzymo-immunoassay, a solid phase constituted of activated cellulose (U.S. patent 3,839,153) is used to render one partner of the binding reaction insoluble. During the determination, some means must be used to keep the sensitized cellulose constantly in suspension. It is also known that the walls of plastic tubes can be sensitized and then used as the solid phase in diagnostic determinations (U.S. patent 4,016,043). In this case an instrument is needed to keep the solution in motion during the determination.

The detection and titration of antigens and antibodies visualized by an aggregation phenomenon of a sensitised solid carrier such as erythrocytes, bentonite, collodium, quartz, synthetic resins and latex, is mentioned in U.S. patent 3,826,613 and U.S. 3,553,310.

These carriers are subject however to various limitations. The preparation of stabilized erythrocytes is
usually difficult and the red blood cells prepared and
sensitized by the methods described in the above
mentioned inventions do not aggregate on a slide in the
presence of antiserum. Chromic chloride used for the
sensitisation of red blood cells allows a visualisation
on a porcelaine tile of the aggregation provided by
specific antiserum, but the sensitisation itself is
transient: the sensitized cells hold for only a week.
No slide test based on the use of sensitized erythrocytes has been marketed up till today. Other carriers
(bentonite, latex, collodium, quartz) which readily
adsorb proteins or polysaccharides aggregate on a
slide in the presence of antiserum but they will also
adsorb spontaneously interfering proteins, antibodies
and polysaccharides present within the fluid to be
analyzed. The use of these carriers brings about a
high incidence in false results. This is implicit in
U.S. patent 3,826,613 where aggregates of latex
particles that spontaneously form with a virus or with
an antibody or other protein serve as an index of
concentration of these substances in the analyzed
fluid. Very clean material containing only the agent
one wishes to analyze, therefore, needs to be used for
the determinations. In addition, these synthetic
solid phases, in order to be acceptable, must meet
rigid requirements in size and quality which are not
always reproducible.

In the present invention use is made of bacteria,
yeast cells, red blood cells as solid carriers for
proteins, polysaccharides, nucleic acids, lectins,
antigens, antibodies immunogens haptens and the like.

Bacteria are easy to grow and can be readily produced in large quantities. Their size, when the bacterium strain and type is well chosen, is very reproducible and their membrane is extremely resilient and fairly inert, that is, free of adsorbing contaminants. Further they may, without fear of damage, be dried, frozen, or lyophilized for storage purposes. Yeast cells are industrially produced and red blood cells may be obtained in large quantities at slaughterhouses. Glutaraldehyde is used for the activation.

The step used to activate the bacteria, yeast cells, red blood cells and other before sensitization also serves to simultaneously fix their condition but, if need be, fixing may take place in a separate earlier step either by formolinisation or by heat shock or by the use of some other bactericidal technique known in the art.

After washing off excess reagents the activated unicellular entities are sensitized. The sensitized solid phase may be employed in a variety of diagnostic determinations based on the interaction of binding partners. For example, it may be used as a slide test. In this case a drop of antigen-sensitized solid carrier is mixed on a glass-slide with a drop of specific antiserum or antigen receptor and a drop of test reagent containing an unknown amount of antigen. After 2-3 minutes the glass-slide is viewed as a transparency against a dark background. In the absence of free antigen, the beads will aggregate in the presence of antiserum. In the presence of free antigen the antiserum activity will be blocked and there will occur an inhibition of aggregation. The same aggregation phenomenon can be used to determine the level of an unknown antiserum.

Also if the yeast cells, red blood cells or bacteria have been sensitized with specific antibodies or a specific receptor, then aggregation is obtained by the admixture of antigen or binding partner. In that case inhibition of aggregation results from the presence of excess free antiserum.

If aggregates obtained by the interaction of a sensitized carrier and its free binding partner are too thin or weak to be detected by the naked eye, they may be detected by more refined means, such as a microscope, a Coulter Counter, a nephelometer, a photometer, a turbidimeter or a light scattering sensing device such as the one developed in U.S. patent 3,905,767.

When appropriately chosen, bacteria have a size that for each bacterial strain remains confined within well defined limits. Uniformity of size is very important when a sensitized solid carrier is used whose aggregation under the influence of a specific binding partner is recorded in a Coulter Counter, in a nephelometer or turbidimeter or when the sensitized bacteria are used as a marker in cytochemistry to detect antigens in histologie slides.

Further bacteria sensitized by a binding partner are useful as solid phases in enzymo-immunological, fluoro-immunological and radio-immunological tests. They present the advantage over other beaded solid carriers of extreme smallness. Their sedimentation rate is therefore so slow that they remain in suspension during the time needed to make a determination and no agitating device is thus required to keep them in suspension, contrary to beads of cellulose, of Sephadex or of polyacrylamide. In addition, they have the advantage over other solid carriers of showing a very low non-specific adsorption of foreign proteins.

Sensitized bacteria or sensitized red blood cells prepared according to the description of the invention are very valuable for use as solid carriers for the inoculation of antigens and haptens into animals. By attachment of the antigen to a solid phase, this antigen is made insoluble and less rapidly eliminated through the subject's kidneys. Usually this result is achieved by including the antigen in droplets of water-in-oil emulsion (Freund incomplete adjuvant); the oil of such emulsion may also contain bacteria (Bordetella Pertussis, Mycobacterium Tuberculosis, Mycobacterium Butyricum) which act to mobilize unspecifically the inmunological defenses of the organisms (Freund complete adjuvant). The inoculation into animals of antigen included in a Freund complete adjuvant or Freund incomplete adjuvant leads many times to the development of uncontrollable sterile abscesses. The occurrence of such abscesses is unacceptable when the vaccine inoculation proceeds in humans or else in farm animals destined to the market since these abscesses mar the meat.

The direct attachement of an hapten or an antigen to bacteria obviates the need for water-in-oil emulsions. Furthermore the bacterial solid carrier may be chosen to be either a strong immunogen such as B. Pertussis, V. Cholerae or M. Butyricum or may be chosen from bacterial strains that have strong interest as a vaccine. (Vibrio Cholerae, Staphylococcus Aureus, Neisseria Gonorrhoaea, etc.). In this case the unspecific immunological effect of the bacteria is further enhanced by incubating the sensitized bacteria in the presence of a soluble adjuvant (N. acetyl-muramyl-L-alanyl-D-Isoglutamine) or double stranded polynucleotides combined to protamine or histones that attach to remaining free activating residues. If the quality of the inoculated meat is of no importance, that is, is not destined to market or for use in human beings or pet animals, these carriers may be further incorporated in a water-in-oil emulsion in order to amplify even more the wanted immunologic response.

The attachment of the hapten or immunogen may also occur on red blood cells originating from the same animal species one uses for the production of antibodies. In this case no foreign material except the attached hapten or immunogen is introduced into the animal and one minimizes thus the creation of unwanted antibodies. Such a system of immunization has been described (see : Journal of Immunological Methods, 23 (1978) 91-97). The coupling agent used to attach hormones to red blood cells is tannic acid which is notoriously poor for this purpose, in that only a fraction of the added hormone will be attached to the cells, the rest remaining unattached and lost during purification steps.

Bacteria are very easy to grow and may be obtained in large quantities at minimal expense. After growth is achieved, the bacteria are collected by centrifugation (e.g. 8,000 rpm for 20 min.) washed once in saline solution and killed or fixed. This killing is accomplished by the standard means at the disposition of the bacteriologist such as ozone bubbling, formalin, heat shock, acrolein, etc.

Yeast cells are obtained from industrial plants and red blood cells are obtained from laboratory animals or slaughterhouses.

Bacteria would be a material of choice for the development of solid carriers useful in diagnostic tests. Yet they are extremely difficult to activate. One approach consists in the use as solid carrier of a bacterium that possesses naturally a specific binding partner on its surface. Such a bacterium exists. It is Staphylococcus Aureus that bears on its surface a protein A. This protein A has the property to react with and bind human gamma globulins. The system has been exploited (U.S. patent 4,189,466 Ainis et al., 1980). Yet, the system is restricted solely to the detection of gamma-globulins and their derivatives. It lacks flexibility.

Likewise, yeast cells, red blood cells and other unicellular organisms (e.g. euglena viridis, blue algae) could fulfil the purpose of serving as a solid carrier. Up till now red blood cells have been widely used. Yet the red blood cells are so poorly sensitized that they can be used solely in haemagglutination tests where the cells are left sedimenting 1 to 2 hours before a result can be obtained. There exists no slide tests based on the aggregation of sensitized red blood cells that could be read in a few minutes, with the exception of chromic chloride sensitized cells. In this case, however, the cells hold for only a few days.

A wider use of bacterias as solid carriers and the use of unicellular animal and plant cells in aggregation tests depends primarily on the evolvement of an efficacious system of activation and sensitization that would attach on the membranes of the cells sufficiently large quantities of a sensitizing compound to make them fully reactive in the presence of antiserum. This has not been up till now the case.

In most sensitisation procedures applied to red cells, only a tiny fraction of the added sensitizing agent is really fixed on the cells. The largest part of it is lost in the washing fluids.

The chemical coupling of sensitizing agents to bacteria, yeast cells red blood cells has been described in various patents among which one should cite U.S. patent 3,553,310 delivered to Czismas in 1971 as well as Patel (U.S. 3,882,225). The granted patents cover almost exhaustively the different coupling agents existing. The examples given range from bisdiazotated benzidine to di-fluoro dinitrobenzene, acroleine, glutaraldehyde, carbodiimide, hydroquinone and cyanogen bromide. These coupling agents were all assayed by us in accordance with the descriptions given by the various authors in the examples given in the patents and also in the demand of Maes.

Not one single coupling system has given satisfaction for the purpose intended : namely be able to activate and couple sensitizing agents to stabilised bacteria, stabilised yeast cells, stabilised red blood cells in such a way that these unicellular organisms fully react by aggregation within a few minutes under the influence of suitable amounts of the corresponding antibodies.

In some cases (hydroquinone (Avrameas, Scand. J. Immunol. 8, 21, (1978) acrolein (U.S. 3,553,310) bisdiazotated benzidine (U.S. 3,882,225), cyanogen chloride (German Of en. 2,126,766) no sensitization could be demonstrated in using bacteria. This failure to sensitise the activated membranes occurred not only with soluble antigen but occurred also when the sensitizing protein was polymerized on itself, as described in U.S. patent 3,882,225. Glutaraldehyde applied in accordance to the examples given (4°C or R.T. applied during a time extending from 2 hours to 18 hours, using also distilled glutaraldehyde) gave erratic results (U.S. 3,553,310 et U.S. 4,223,005). It sometimes worked, sometimes not at all. This is also described in "techniques in clinical immunology" second edition, 1981, p. 67. We also tried polymerized glutaraldehyde (Macromolecules 13, 19-23 (1980) in the conditions described by the authors and have consistently failed in our endeavours to sensitize bacteria, yeast or red blood cells so heavily that they would become of value for aggregation tests or as carriers for affinity chromatography). Carbodiimide used according to U.S. patent 4,100,268 July 11, 1978, gave also negative results. No diagnostic tests based on the use of bacteria or red cells in a diagnostic slide test is yet available.

Surprisingly, according to the invention it has been discovered that glutaraldehyde may be used for this purpose, when applied in an as yet undescribed manner.

More specifically, according to the invention, the activation of a bacterial strain, yeast cells, red blood cells or other unicellular living entities is performed with an activating agent essentially consisting of a glutaraldehyde solution at a final concentration of at least 2.5% mixed with one of said bacterial strain, yeast cells, red blood cells or other unicellular entities.

The activation step can be performed at room temperature but is preferably performed at a temperature of at least about 37°C or even higher if the material withstands the heat and the reaction is allowed to proceed during at least 24 hours, preferably at least 48 hours.

According to a preferred embodiment, the temperature ranges from 20°C, preferably about 37°C, to 60°C during at least 24 hours, preferably at least 48 hours.

The pH range was found not to be very important but should be initially between 5 and 9.

According to another advantageous embodiment, said bacterial strain, yeast cells, red blood cells or other unicellular entities are under the form of a suspension at about 20% (volume/volume) in buffered saline preferably phosphate buffered saline.

According to another advantageous embodiment of the invention method, the sensitization is performed by mixing a suspension of the activated bacterial strain, yeast cells, red blood cells or other unicellular entities at about 50% (volume/volume) with the sensitizing agent and reacting the mixture thus obtained under agitation at a pH ranging from 5 to 10 during at least 24 hours at about 37°C or more.

Preferably, the sensitizing agent is added either as a dry powder or else as a concentrated solution.

After sensitization has taken place, the products are preferably thoroughly washed and stored at 10% (volume/volume) at 4°C under a protecting agent such as merthiolate, sodium azide or formaldehyde.

Sensitized bacteria possess a vast range of interesting applications not shared by other solid carriers.

Compared to other beads, they are very small and present an enormous surface of exchange. They are sufficiently small and light so that they do not sink to the bottom of reacting tubes during manipulations and determinations, yet sufficiently dense to be easily collected by centrifugation. Their membrane is very tough and resilient, may be submitted to various treatments without fear of destruction, yet is also fairly inert so that they do not spontaneously adsorb unspecific contaminants. This is very important in diagnostic determinations of small quantities of substances.

Bacteria may also be stained. They may be coloured in blue by methylene-blue, Turks stain or May-Grunwald stain. Pyronin will colour them red. Gram stain will colour them according to their classification. Sensitized stained bacteria provide an excellent method to detect agglutination in the presence of specific antiserum. When put on a glass-slide at a suitable concentration, 10 µl of bacteria sensitized with a binding partner + 10 µl of antiserum or antigen well mixed will produce in 3 minutes a visible agglutination. In the presence of an additional 10 µl of fluid containing the free binding partner similar to the partner attached to the bacteria, an inhibition of agglutination will take place. Surprisingly also, identically sensitized red blood cells aggregate in a visible way in slide tests, which is not the case when other coupling agents are used, yet they become much more reactive still when their specific agglutination is promoted by sensitized bacteria. The visualization of the agglutination is then enhanced in a very striking way.

Agglutination is detected not necessarily only by visual inspection on a glass-slide. It may also be
detected after slow sedimentation to the bottom of a
test tube or can be detected by aggregate counting in
a Coulter Counter or by light intensity changes in a
nephelometer or turbidimeter or by light scattering.
Since bacteria are very uniform in size, sensitized
by a covalent bondage, very solid and little influenced by the presence of extraneous contaminants,
their use in nephelometry or in aggregate counting by
a Coulter Counter is more rewarding than the use of
latex particles or other solid carriers. Also, the
aggregation of latex particles by proteins occurs
spontaneously in an uncontrollable way, whereas the
bacteria are preliminarily sensitized by the sensitizing substance using covalent bondages and the degree
of their sensitization may be easily controlled by
varying the amount of sensitizing substance or the
amount of aggregating free binding partner.

Very small organisms like Bordetella Pertussis stained
by M. Grünwald are preferred when sensitized bacteria
are used in cytochemistry as markers to locate the
presence of a binding partner on a tissue slide.
These sensitized bacteria easily recognized may also
be enzymatically or fluorescently tagged for better
recognition and are an excellent substitute to conventionally enzyme-labelled or fluorescent-labelled
proteins.

The very small size of the sensitized bacteria offering a comparatively great surface area, together with
the absence of adsorption of unspecific proteins as
well as their slow sedimentation rate make them
valuable in diagnostic tests where a solid phase is
used.

In radio-immunological, enzymo-immunological and fluoroimmunological methods based on the use of a sensitized solid phase destined to isolate one of the binding partners specifically involved in a binding reaction, it is essential to reduce as much as possible disturbing unspecific adsorptions. Also, the need to keep the solid phase perpetually in suspension or else the need to perpetually keep the liquid phase in motion when the solid phase consists in the walls of the reacting tube, is bothersome and costly. When bacteria are used as a sensitized solid phase, these two difficulties are avoided. The bacteria do not sediment during the time needed for the performance of most determinations (24 to 48 h) and further, their inertness to the presence of foreign proteins in the test fluids allows the use of larger amounts of this fluid and increases the sensitivity of the system.

In addition to this, the enormous surface of exchange they provide allows the use of only minute amounts of them in each determination. This substantially reduces the costs of each determination.

Sensitized bacteria may serve as immunologic adjuvants capable of enhancing the immune response upon inoculation. The creation of the antibodies against haptens or other wise immunologically inert molecules, such as nucleotides, nucleic acids, steroids or biogenic amines, is usually achieved by coupling them to immunogenic proteins such as bovine gamma-globulins, bovine albumin or other. The same purpose may be achieved with much greater efficacy by attaching the hapten to the strong immunogen that is bacterium. When the bacteria used as solid phase are properly chosen (B. Pertussis, V. Cholerae, M. Butyricum, etc) the hapten becomes a very strong immunogen itself, whose ability to mobilize the totality of the immunological defense system of the inoculated organism is further increased by coupling to the remaining activated groups of the sensitized bacteria, additional immunological adjuvants such as N-acetyl-muramyl-L-alanyl-D. Isoglutamine or else poly (I:C) or poly (A:U) coated with protamine or histone. Also, some bacteria may be chosen which hold definite interest as vaccines, such as V. Cholerae or B. Pertussis.

The immunogen is then almost at its maximum of efficacy. If need be, further enhancement of the immunological potency of the vaccine may be obtained by including the immunogen within droplets of water-in-oil emulsion.

Red blood cells activated and sensitized in the same way may be used in the same manner for the obtention of large amounts of antibodies.

The following examples serve to illustrate clearly and fully the practice of the invention but are not to be regarded as limiting : (percentages are given by weight unless otherwise stated, or inappropriate).
Example 1

0.2 ml of E. Coli were mixed with 0.2 ml of phosphate buffer 0.2 M at pH 7.5 and activated with 2.5% glutaraldehyde for 48 hours at 50°C. The bacteria turned orange upon activation. Sensitization with HCG (2 mg for 1 ml of 50% suspension of bacteria in buffer at pH 8) proceeded during 48 h at 37°C. After washing the suspension was used in a slide test for the detection of HCG.

Ten microliters of the sensitized suspension were mixed on a glass slide with 10 µl of anti-HCG antiserum diluted to 1.400 in EDTA buffer and 10 µl of urine containing various concentrations of HCG ranging from 62.5 IU/liter to 1000 IU/liter were added. After thorough mixing, the slides were tilted for 3 min. at RT after which they were visually inspected to detect agglutination or inhibition of agglutination. Agglutination of the bacteria occurred until a concentration of 250 HCG in the urine was present. At higher concentrations of HCG an inhibition of agglutination took place. This test is thus very sensitive and extremely competitive with slide tests based on the use of latex whose limit of sensitivity is about 2000 - 3000 IU/liter.

Example II

E. Coli was sensitized with HCG as in Example I. After washing, the bacterial suspension was mixed at a 1:50 dilution with a 20% suspension of HCG-sensitized sheep red cells, in EDTA buffer pH 7.5 (1.7 gr/ 100 ml). Ten microliters of the double suspension was added to the 10 µl of a working dilution of antibodies and to 10 µl of different urines containing various amounts of HCG. After spreading over a glass slide, the suspension was tilted for 3 min. at RT, then visually inspected for agglutination. Agglutination of the red cells occurred until the concentration of HCG in the tested urines reached a level of 500 IU/1. At higher concentrations of HCG, agglutination was inhibited. The agglutination, furthermore, was very VISIBLE. This pregnancy slide test is thus very easy to read and very sensitive. It compares well with similar latex tests.

Example III

Staphylococcus aureus was activated by glutaraldehyde
as in Example I and sensitized with rubella antigen.
After sensitization it was stored as a 10% suspension
in EDTA buffer pH 7.5 (1.7 gr/100 ml). The limit of
dilution of rubella positive antiserum was found to
be 1:4000 when mixed in equal volumes with a 1:50
dilution of the antigen sensitized solid carrier.

Further dilutions of the antibody resulted in a
failure of the mixture to show any distinctive
agglutination under visual inspection on a glass slide
after 5 min. incubation at RT. The same suspension
of rubella sensitized bacteria was tested for
agglutination in a Coulter Counter. The suspension
was diluted 1:20.000 in Isoton pH 7.4. A sample
containing antibodies was added to an exactly determined volume of the bacterial suspension and incubated
over 30 min. at 37°C.

Coulter Counter (Model F from Coulter Electronics, Inc.,
Hialesh, Fla) settings were : aperture 16, attenuation
1, orifice diameter 70 u and threshold value 0. All
settings throughout the analysis remained unchanged
except for the threshold value which was varied. At
higher threshold levels, the machine registers only
larger aggregates and one can thus obtain an analysis
of the aggregate pattern which occurred due to the
addition of the antibody.

Three vials were prepared, of which N°. 1 contained
no antibody, N°. 2 contained antibody at a dilution
of 1:16.000 and N°. 3 contained antibody at a
dilution of 1:400. Three counts were made each time
at all threshold values applied and the average of the counts is given in Table I.

## Table 1

| vial number threshold value | 1 | 2 | 3 |
|---|---|---|---|
| 0 | 68.872 | 30.884 | 15.654 |
| 1 | 35.189 | 26.493 | 14.695 |
| 2 | 15.062 | 20.447 | 12.752 |
| 3 | 2.127 | 12.654 | 10.862 |
| 4 | 961 | 6.992 | 10.694 |
| 5 | 460 | 3.654 | 8.253 |
| 6 | 454 | 2.541 | 7.032 |

1 : control Rubella-sensitized bacteria

2 : anti-Rubella antiserum 1 : 16.000

3 : anti-Rubella antiserum 1 : 400

It is clear from the table that aggregates are found not only when a dilution 1:400 antiserum is applied, but also when a higher dilution is applied. This antibody-related aggregation is not any longer visible by eye but still detectable by optical means.

## Example IV

Confluent coverslip cultures of Baby Hamster Kidney cells (BHK) were infected with Rubella virus and incubated at 36°C for 48 hours. The cells were thereafter washed once with phosphate buffered saline and incubated for 1 hour at 37°C with serial dilutions of human serum obtained from cases of acute rubella. The dilutions were made in anti-rubella negative serum. After washing the coverslips now carrying human antibodies to rubella virus they were treated at 37°C for 45 min. with the appropriate working dilution of glutaraldehyde activated bacteria sensitized with affinity purified goat antiglobulins against human IgG, IgM and IgA. A portion of the anti-IgG bacteria batch was further fluorescein labelled.

The coverslips were carefully washed and examined either by illuminated microscope to determine the presence of immune adhering bacteria or else by dark-field illumination in a Reichert microscope. A quartz-halogen light source was used with an interference excited filter and an OG 530 barrier filter. Fluorescein-labelled bacteria exhibited an extremely strong, discrete fluorescence.

Specific immunoglobulin titers were measured in 18 sera obtained at various times from 4 adults with acute rubella.

I Microscopic observation of bacterial presence

All three classes of antibody, as revealed by the presence of bacteria on the coverslips, increased virtually simultaneously within 3 days after the onset of the rash. IgM declined after the second week (maximum titer : 1/512) and could not be detected after 24 days in undiluted serum. IgA displayed a similar type of response. IgG antibody reached a maximum (1:2.048) after about 2 weeks and persisted with slight declination for 150 days, after which the experiment was stopped.

II Immunofluorescence allowed a 2 to 4 fold increase in sensitivity, in that a recognition of the presence of bacteria was much facilitated by the observation of

discrete blobs of fluorescent staining which were absent from the controls.

Example V

0.5 ml of E. Coli were diluted in 5 ml of 0.1 M phosphate buffer pH 7.0 and activated with 2.5% (final) glutaraldehyde during 48 hours at 50°C. After washing the cells were sensitized with alpha-foeto-protein (aFP) - (Behring Werke). The sensitized bacteria were treated further with hydroxylamine in order to eliminate the unreacted glutaraldehyde residues and were used in an enzyme-immunological test for the detection of aFP.

One milliliter samples of rabbit antibodies against aFP were incubated in separate tubes for 3 hours at 37°C with 50 $\mu$l of serum containing known amounts of aFP, ranging from 5 ng/ml to 250 ng/ml. After incubation the different reaction tubes were completed with 50 $\mu$l of a suspension of aFP-sensitized bacteria and the tubes incubated further during 18 hours at RT.

The bacterial suspension was then centrifuged down, resuspended in 1 ml of sheep antiglobulins against rabbit globulins, labelled with peroxidase. After 2 hours incubation at RT in this medium, the bacterial suspension was collected by centrifugation, washed once with cold tap water and the peroxidase attached to the suspension was detected by means of ortho-phenylene diamine in citrate buffer pH 5.0. Colour development was stopped after 30 min. incubation at RT by addition of 9M $H_2SO_4$. Adsorbance was measured at 480 nm.

This system made possible the detection of 5ng/ml aFP present in the analyzed serum. This limit of sensitivity is very low.

Example VI

The antigen or antibody-coupled bacteria may be easily adhered to object slides with gelatin. These slides can be handled as histologic preparations on which immunohistochemical procedures can be performed. All that is then needed for serodiagnostic purposes is an illuminated microscope and staining jars. Object slides were cleansed in a 100% alcohol-ether mixture (1:1) and dipped in a suspension of HCG-coupled bacteria via GA obtained as in Example I in PBS containing 0,1% gelatin, which was dried for 2 hours in air. Rabbit anti-HCG antiserum was incubated at RT for 1 hour in the presence of various HCG concentrations. A drop of the reaction mixtures was placed on the bacteria-slide and further incubated for 1 hour. The slides were rinsed with tap water and incubated with a HRP-conjugated goat antirabbit IgG solution for 1 hour. After the incubations, the slides were rinsed, stained with 0.05% 3-3' diam no-benzidine tetrahydrochloride (DAB) and 0.05% $H_2O_2$ in 0.05 M citrate buffer at pH 4.0 and studied either by visual inspection or under the microscope. In the absence of HCG antigen, the spots on the slides were coloured deep brown. In the presence of 1.600 IU/liter antigen, the spots on the slides were barely coloured. The smallest amount of antigen that produced a difference in the intensity of the coloration observable by eye was 50 UL/liter HCG.

Example VII

Commercially obtained Bordetella Pertussis was sensi-

tized with the $\beta$-subunit of HCG by incubating 0.8 ml of glutaraldehyde activated bacteria obtained as in Example I with 0.8 ml of phosphate-buffered saline at pH 8.0 containing 400 $\mu$g/ml B HCG for 48 hours at 37°C. After sensitization the bacteria were further incubated with 1 mg of the water-soluble immunity adjuvant N-acetyl-muramyl-L-analyl-D- Isoglutamine for 18 hours at RT.

The sensitized and immunology-enhanced bacteria were then emulsified in a water-in-oil emulsion and inoculated by doses of 0.4 ml in multiple intradermal sites in rabbits. Five rabbits received the sensitized bacterial suspension and five rabbits received the same ingredients emulsified in oil but not covalently coupled to the bacteria which were not activated.

The rabbits were bled 35 days later and the level of anti-HCG antibodies present was assessed through a test using sensitized sheep erythrocytes. The maximal dilution of the individual antiserum that could be made and still agglutinate the sensitized red cells is recorded in Table II.

## Table II

| Control Rabbits | Experimental Rabbits |
|---|---|
| 1 : 32.000 | 1 : 128.000 |
| 1 : 16.000 | 1 : 64.000 |
| 1 : 2.000 | 1 : 16.000 |
| 1 : 16.000 | 1 : 64.000 |
| 1 : 16.000 | 1 : 32.000 |

The data from Table II indicate that the covalent coupling of the antigen to the bacteria incorporated into the Freund complete adjuvant and further treated with a soluble immunological adjuvant, is beneficial in the production of higher-titered antibodies.

Example VIII

Sheep red blood cells collected at the slaughterhouse were washed in saline. A 20% suspension of sheep RBC in PBS was mixed 1:1 with a 5% solution of G.A. and put under agitation at 37°C during 3 days. The cells were washed and sensitised with HCG at 37°C during 2 days, as in Example I. The cells aggregated in the presence of antibodies on a slide test and could further be used as immunogen for the creation of antibodies in rabbits.

Preferably the glutaraldehyde solution is at a final concentration of glutaraldehyde ranging between about 2.5% and about 10%, preferably 5%.

Claims

1. A method of sensitizing a bacterial strain, yeast cells, red blood cells or other unicellular living entities which comprises the step of activating one of a bacterial strain, yeast cells, red blood cells or other unicellular entities;and reacting the same with a sensitizing agent of the group essentially consisting of an antigen, an antibody, a hapten, a nucleic acid, a carbohydrate, an aminoacid, an affinity receptor and a protein, to produce the bacterial strain, yeast, red blood cells or other unicellular entities, having the sensitizing agent coupled thereto, characterized in that the activating step is performed with an activating agent essentially consisting of a glutaraldehyde solution at a final concentration of at least 2.5% mixed with one of said bacterial strains, yeast cells, red blood cells or other unicellular entities at a pH ranging from 5 to 9, at a temperature ranging from 20°C to 60°C and during at least 24 hours, preferably at least 48 hours.

2. A method according to claim 1, wherein one of said bacterial strains, yeast cells, red blood cells or other unicellular entities are under the form of a suspension at about 20% (volume/volume) in buffered saline, preferably phosphate buffered saline.

3. A method according to claim 1 or 2, wherein the temperature is at least about 37°C and the reaction proceeds during at least about 48 hours.

4. A method according to claim 1, 2 or 3, wherein sensitization is performed by mixing a suspension of the activated bacterial strain, yeast cells, red blood cells or other unicellular entities at about 50%
(volume/volume) with said sensitizing agent and
reacting the mixture thus obtained under agitation
at a pH ranging from 5 to 10 during at least 24 hours
at about 37°C or more.

5. A method according to any one of claims 1 to 4,
for the detection and determination of a component of
an antigen-antibody reaction and of a receptor-binding
partner reaction in a liquid sample containing the
component to be determined, comprising the step of
providing a reagent consisting of a solid phase of
one component of said reaction selected from the
group consisting of an antigen, an antibody and a
receptor covalently bound to the surface of a bacterial strain, yeast cells, red blood cells or unicellular entities, contacting a given quantity pf
said liquid sample with said reagent and determining
the extent of aggregation of the solid phase as a
measure of the presence and quantity of the component
to be determined.

6. A method according to any one of claims 1 to 4,
for the detection and determination of a component of
an antigen-antibody reaction and a receptor-binding
partner reaction on a solid surface, wherein one of
the components of the reaction is immobilized on a
solid surface, comprising the step of providing a
given quantity of a reagent of an antigen, an antibody, an antiglobulin and a receptor covalently
bound to the surface of a bacterial strain and having
binding properties for said component immobilized on
the solid surface, contacting said solid surface with
said reagent, washing the solid surface and determining the extent of the presence therein of the bacteria
immunologically or affinity-bound to it as a measure of the presence and quantity of the component to be determined.

7. A method according to any one of claims 1 to 4, for the detection and determination of a component of a binding partners reaction on a solid surface, comprising the step of immobilizing on a solid surface a given quantity of a reagent consisting of one component of said reaction selected from the group consisting of an antigen, an antibody, an antiglobulin and a receptor covalently bound to the surface of a bacterial strain, contacting it with a liquid sample containing a binding partner for the component immobilized on the solid surface and determining the extent of binding partner immunologically or affinity attached to the bacterial phase immobilized on the solid surface.

8. A method according to any one of claims 1 to 4, for the isolation and determination of a component of an antigen-antibody reaction and of a receptor-binding partner reaction in a liquid sample containing said component comprising the steps of :
    a) contacting the liquid sample with a reagent comprising a solid phase consisting of one component of said reaction selected from the group consisting of an antigen, an antibody, an antiglobulin, a lectin and a receptor covalently bound to the surface of a bacterial strain, yeast cells, red blood cells and other unicellular entities.
    b) isolating the solid phase, and
    c) determining the quantity of binding partner attached to the solid phase.

9. A method according to any one of claims 1 to 4, for the production of antibodies comprising the steps of :

a) sensitizing a bacterial strain, red blood cells, yeast and other unicellular entities, with a component selected from the group consisting of an antigen, an immunogen and a hapten, and

b) inoculating the sensitized carrier into an animal or a human.

10. The methods of claims 5 and 6 wherein bacterial coagglutination with another naturally reactive or sensitized particulate solid phase is taking place.

11. The methods of claims 5, 6 and 10, wherein the sensitized carriers are stained.

12. The methods of claims 5, 6, 10 and 11, wherein the aggregation is evaluated by a slide test, by nephelometry, by turbidimetry, by aggregate size evaluation, by visual inspection, by microscopy or by light scattering.

13. The methods of claims 5, 6, 7 and 8, wherein the sensitized solid phase isotopically, enzymatically or fluorescently tagged.

14. The methods of claims 5, 6, 7 and 8, wherein the sensitized solid phase is further contacted with an isotopically, enzymatically or fluorescently tagged binding partner for the sensitized solid phase or an attached part thereof.

15. The methods of claim 8, wherein the solid reaction phase is eluted and isolated by acid treatment, chaotropic treatment, alkali treatment or competitive affinity treatment.

16. The method of claim 8, wherein the quantity of binding partner immunologically or affinity attached to the sensitized solid phase is determined by providing simultaneously or at a later time a second reagent having binding properties for one of the binding partners covalently, immunologically or affinity bound to the solid phase, said second reagent being tagged by a radioisotope, a fluorophore and an enzyme.

17. The method of claim 9, wherein the sensitized bacterial strain, yeast cells, red blood cells and other unicellular entities are further covalently linked to a soluble unspecific immunological adjuvant, preferably through incubation of a  mixture  of both.

18. The method of claim 17, wherein the immunological adjuvant is N- acetyl-muramyl-L-alanyl-D-Isoglutamine.

19. The method of claims 9, 17 and 18 wherein the sensitized bacterial strain yeast cells, red blood cells are incorporated into the aqueous phase of a water-in-oil emulsion.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 000 102 (AMERICAN HOME PRODUCTS CORP.)<br>* Pages 5,6; pages 12,13; example 2; claims 1,2,4,8,9,11 *<br>--- | 1,2,5, 12 | G 01 N   33/54<br>C 07 G   7/00 |
| Y | EP-A-0 023 431 (BROCADES (G.B.) LIMITED)<br>* Page 3; page 5, line 20 - page 9, line 11; examples 1-3,8,9; claims 1,2,4-6,10 *<br>--- | 1,2,5, 8,11-13 | |
| P,Y | US-A-4 403 037 (S.R. COATES)<br>* Claims 1,2,5,9,12,14,15<br>--- | 1,5,12 | |
| Y | GB-A-2 101 630 (SOUTH AFRICAN INVENTIONS DEVELOPMENT CORP.)<br>* Pages 1-4; page 7, lines 50-65; page 8, lines 18-41; examples; claims 1,2,10,11,13,15,16 *<br>--- | 1,9,17 | TECHNICAL FIELDS SEARCHED (Int. Cl. 3) |
| Y | FR-A-2 345 461 (DAINIPPON PHARMACEUTICAL CO., LTD.)<br>* Whole document *<br>--- | 1,2,8, 14,16 | G 01 N<br>A 61 K<br>C 07 G |
| D,Y | US-A-4 223 005 (M.C. TEODORESCU et al.)<br>* Whole document *<br>--- | 1,2,6, 7,10, 12 | |
| | -/- | | |

The present search report has been drawn up for all claims

| Place of search THE HAGUE | Date of completion of the search 26-04-1984 | Examiner GRIFFITH G. |
|---|---|---|

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. ³) |
|---|---|---|---|
| Y | FR-A-2 380 296 (OTSUKA PHARMACEUTICAL CO., LTD.) * Page 4, line 31 - page 5, line 10; examples; claims 1,5-7 * | 1,2,4 | |
| A | US-A-4 399 229 (A.A. KELTON et al.) | | |
| A | FR-A-2 244 170 (SIBIEM SOCIETE INTERNATIONALE DE PRODUITS BIOLOGIQUES ET MEDICAUX) | | |
| | | | TECHNICAL FIELDS SEARCHED (Int. Cl. ³) |

The present search report has been drawn up for all claims

| Place of search THE HAGUE | Date of completion of the search 26-04-1984 | Examiner GRIFFITH G. |
|---|---|---|